# EUROPÄISCHE PATENTSCHRIFT

(11) **EP 1 117 337 B1**
(45) Veröffentlichungstag und Bekanntmachung des Hinweises auf die Patenterteilung: **01.09.2004**
(21) Anmeldenummer: 99959192.8
(22) Anmeldetag: 30.09.1999
(51) Int. Cl.: A61B 17/72

(54) **MARKNAGEL**
INTERMEDULLARY NAIL
CLOU D'OSTEOSYNTHESE INTRAMEDULLAIRE

(30) Priorität: 02.10.1998 DE 19847253
(43) Veröffentlichungstag der Anmeldung: 25.07.2001
(73) Patentinhaber: aap Implantate AG, 12099 Berlin (DE)
(72) Erfinder: AHRENS, Uwe, D-12107 Berlin (DE); KLOCKOW, Andreas, D-12347 Berlin (DE)
(74) Vertreter: Meissner, Peter E., Dipl.-Ing.
(86) Internationale Anmeldenummer: PCT/DE1999/003238
(87) Internationale Veröffentlichungsnummer: WO 2000/019924

(56) Entgegenhaltungen:
- WO-A-98/34555
- US-A- 5 766 174
- US-A- 5 772 662

## Beschreibung

Die Erfindung betrifft Marknagel mit Schrauben zur Verriegelung des Nagels im Knochen gemäß dem Oberbegriff des Patentanspruchs 1.

Ein derartiger Marknagel ist aus der US-A-5 766 174 bekannt.

Bei Marknägeln ist es somit bekannt am proximalen Ende eine Kappe aufzusetzen. Diese liegt in der Gegend der Knochenöffnung, durch die der Marknagel eingebracht wurde und dient zum einen als Platzhalter für das Ausschlaginstrumentarium. Andererseits werden die senkrecht zur Marknagelachse liegenden Bohrungen vor dem Einwachsen von Gewebe geschützt, so daß das Ausschlaginstrumentarium ohne zusätzliche Arbeitsgänge eingeschraubt werden kann.

Zu den Marknägeln gehört heute auch ein Zielinstrumentarium, mit dem möglichst alle Verriegelungslöcher bzw. -rinnen ohne Röntgenkontrolle sicher besetzt werden sollen. Dem zu Folge hat das Implantat an einem Ende zum Beispiel ein Innengewinde und einen Passungsanteil. Gerade diese wichtigen Zonen verhindern bisher eine, wie am anderen Marknagelende übliche, Verriegelung, die sehr nahe am Nagelende sitzt.

Diesem Umstand kommt immer mehr Bedeutung zu, da z. B. Oberschenkelnägel zunehmend retrograd, d. h. vom Knie, aus eingebracht werden. Die Indikationen sind dabei meist Brüche, die im unteren Knochendrittel lokalisiert sind und zum Teil in den Kondylenbereich hineinragen. Existiert aber die erste Verriegelungsmöglichkeit erst 20 - 30 mm unterhalb des Marknagelendes kann ein solches Implantat nicht verwendet werden.

Besonders bei der Implantation eines Marknagels in den Oberschenkel von der Hüfte aus hat der Operateur keine direkte Sicht auf den Nageleintrittspunkt in den Knochen. Er ist somit auf seinen Tastsinn angewiesen. Dabei kann ihm ein Instrument, daß die Kappe nicht nur drehen, sondem auch noch festhalten kann sehr hilfreich sein. Auch kann ein solcher Mechanismus bei Schrauben und den Nägeln selbst angewendet werden.

Aufgabe der Erfindung ist es, eine Marknagel bereitzustellen, der auch eine Verriegelung am äußersten proximalen Ende des Nagels ermöglicht.

Diese Aufgabe wird erfindungsgemäß bei einem Marknagel mit Schrauben zur Verriegelung des Nagels im Knochen, der mit mehreren Ausnehmungen versehen ist, in die die Schrauben einsetzbar sind und der am proximalen Ende mit einer Halteanordnung für das Einsetzen bzw. Extrahieren des Nagels verbindbar ist, wobei zur Befestigung der Halteanordnung am proximalen Ende des Marknagels ein Außenund/oder ein Innengewinde vorgesehen sind, in das eine mit einem korrespondierenden Gewinde versehene Kappe schraubbar ist und
am äußersten proximalen Ende eine senkrecht zur Marknagellängsachse angeordnete Kerbe vorgesehen ist, dadurch gelöst, dass die Kappe nahe dem Kappengrund eine mit der Kerbe ausrichtbare Durchgangsbohrung aufweist, durch die eine weitere Schraube einbringbar ist.

Weitere Ausgestaltungen ergeben sich aus den Unteransprüchen.

Die Erfindung soll nachfolgend unter Bezug auf die Zeichnung erläutert werden.

Dabei zeigt:
Fig. 1 eine vereinfachte Darstellung des proximalen Endes des Marknagels,
Fig. 2 eine Darstellung der Kappe teilweise geschnitten,
Fig. 3 eine Ausgestaltung der Kappe an der Außenseite,
Fig. 4 eine abgewandelte Ausführung des Marknagels,
Fig. 5 und 6 Einzelheiten der Kappenausbildung.

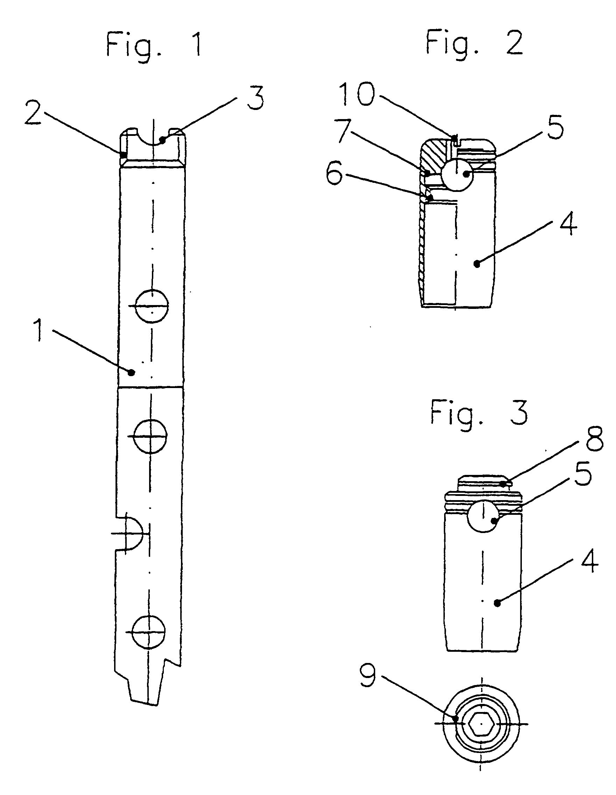

Der Marknagel 1 hat gemäß Figur 1 am Ende das Gewinde 2 und die Kerbe 3. Die Kappe 4 ist mit einer Durchgangsbohrung 5 quer zur Nagel- bzw. Kappeniängsachse versehen und weist ein Innengewinde 6 auf. Der Kappenboden ist mit 7 bezeichnet.

Der Marknagel 1 kann - vor dem Aufschrauben der Kappe - in der üblichen Weise eingebracht und verriegelt werden. Sodann wird das Standardzielinstrumentarium entfernt und durch das Kappenzielgerät ersetzt. Durch dieses hindurch kann nun die Kappe auf den Marknagel aufgeschraubt werden, und zwar soweit, daß der Marknagel kurz vor dem Kappengrund endet oder an diesem anliegt. Das Kappenzielgerät gibt die Winkellage der Kappe im Verhältnis zur proximal äußersten Kerbe so vor, daß eine Fehlstellung ausgeschlossen ist. Über die im Zielbügel des Zielgerätes angebrachte Bohrung kann anschließend das Verriegelungslcch gebohrt und die Schraube eingebracht werden. Diese liegt dann am äußersten proximalen Ende des Marknagels, so daß auch dort eine sichere Verriegelung gewährleistet ist.

Die Kappe kann an der Oberseite ein Formelement 9 oder 10 aufweisen, mit dessen Hilfe eine eindeutige Positionierung des Kappenzielgerätes möglich ist.

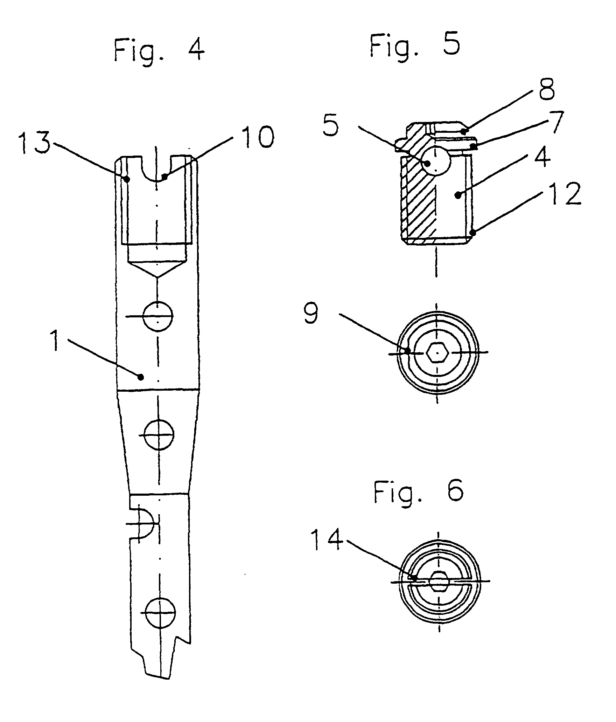

Aus der Figur 3 ist ersichtlich, daß die Kappe auch einen Aufsatz oder Ansatz aufweisen kann, der einen Hinterschnitt 8 besitzt. Dieser Hinterschnitt 8 ermöglicht es in Kombination mit der entsprechenden Anpassung des Ziehgerätes, daß die Kappe nicht nur gedreht sondern auch festgehalten werden kann. Die Figuren 3, 5 und 6 zeigen unterschiedliche Ausbildungen der Kappe an der Außenseite, und zwar mit einer Abflachung 9 bzw. einer Ausnehmung 14. Beides dient zur Positionsvorgabe des Zietinstnjmentariums bzw. zum Aufschrauben der Kappe.

## Patentansprüche

1. Marknagel mit Schrauben zur Verriegelung des Nagels im Knochen, der mit mehreren Ausnehmungen versehen ist, in die die Schrauben einsetzbar sind und der am proximalen Ende mit einer Halteanordnung für das Einsetzen bzw. Extrahieren des Nagels verbindbar ist, wobei zur Befestigung der Halteanordnung am proximalen Ende des Marknagels ein Außen- und/oder ein Innengewinde vorgesehen sind, in das eine mit einem korrespondierenden Gewinde (6) versehene Kappe (4) schraubbar ist und am äußersten proximalen Ende eine senkrecht zur Marknagellängsachse angeordnete Kerbe (3, 10) vorgesehen ist,
**dadurch gekennzeichnet,**
**dass** die Kappe (4) nahe dem Kappengrund (7) eine mit der Kerbe (3) ausrichtbare Durchgangsbohrung (5) aufweist, durch die eine weitere Schraube einbringbar ist.

2. Marknagel nach Anspruch 1,
**dadurch gekennzeichnet,**
**daß** die Kappe (4) ein Innengewinde (6) und der Marknagel ein Außengewinde (2) aufweist.

3. Marknagel nach Anspruch 1,
**dadurch gekennzeichnet,**
**daß** die Kappe einen Außengewindestift (12) und der Marknagel ein Innengewinde (13) aufweist.

4. Marknagel nach einem der vorstehenden Ansprüche,
**dadurch gekennzeichnet,**
**daß** die Kerbe (3) und entsprechend die Durchgangsbohrung (5) oval, n-eckig oder kreisförmig ist.

5. Marknagel nach einem der vorstehenden Ansprüche,
**dadurch gekennzeichnet,**
**daß** an der Kappe, und zwar am äußeren Kappenboden, ein Aufsatz oder Ansatz vorgesehen ist, der eine Hinterschneidung (8) aufweist.

6. Marknagel nach einem der vorstehenden Ansprüche,
**dadurch gekennzeichnet,**
**daß** an der Oberseite der Kappe ein Formelement (9,10) zur eindeutigen Positionsvorgabe für ein Zielinstrumentarium bzw. zum Aufschrauben hat.

7. Marknagel nach einem der vorstehenden Ansprüche,
**dadurch gekennzeichnet,**
**daß** die Kappe (4) an der Oberseite eine Ausnehmung (14) in Form einer Nut zur Positionsvorgabe für ein Zietinstrumentarium bzw. zum Aufschrauben der Kappe (4) auf den Marknagel hat.

## Claims

1. An intermedullary nail with screws for locking the nail in the bone, which is provided with a plurality of recesses into which the screws can be inserted and which can be connected at the proximal end to a holding arrangement for inserting and extracting the nail, wherein an external and/or an internal thread is provided for fastening the holding arrangement at the proximal end of the intermedullary nail, into which thread a cap (4) provided with a corresponding thread (6) can be screwed and a notch (3, 10) arranged at right-angles to the longitudinal axis of the intermedullary nail is provided on the outermost proximal end,
**characterised in that**
the cap (4) close to the cap base (7) has a through-bore (5) which can be aligned with the notch (3) and through which a further screw can be introduced.

2. An intermedullary nail according to Claim 1, **characterised in that** the cap (4) has an internal thread (6) and the intermedullary nail an external thread (2).

3. An intermedullary nail according to Claim 1, **characterised in that** the cap has an externally threaded pin (12) and the intermedullary nail has an internal thread (13).

4. An intermedullary nail according to one of the preceding claims, **characterised in that** the notch (3), and correspondingly the through bore (5), is oval, n-cornered or circular.

5. An intermedullary nail according to one of the preceding claims, **characterised in that** a projection or attachment which has an undercut (8) is provided on the cap, namely on the outer cap base.

6. An intermedullary nail according to one of the preceding claims, **characterised in that** [it] has a shaped element (9, 10) for clearly setting the position for a targeted instrument set or for screwing-on on the upper side of the cap.

7. An intermedullary nail according to one of the preceding claims, **characterised in that** the cap (4) has on its upper side a recess (14) in the form of a groove for setting the position for a targeted instrument set or for screwing the cap (4) on to the intermedullary nail.

## Revendications

1. Clou médullaire, avec vis pour le verrouillage du clou dans l'os, qui est pourvu de plusieurs évidements dans lesquels les vis peuvent être introduites et qui peut être relié à l'extrémité proximale à un agencement de retenue pour l'introduction ou l'extraction du clou, un filetage mâle et/ou femelle étant prévu pour la fixation de l'agencement de retenue à l'extrémité proximale du clou médullaire, dans lequel filetage un capuchon (4) pourvu d'un filetage correspondant (8) peut être vissé et dans lequel il est prévu à l'extrémité proximale la plus extérieure une entaille (3, 10) agencée perpendiculairement à l'axe longitudinal du clou médullaire,
**caractérisé en ce que** le capuchon (4) présente à proximité de la base de capuchon (7) un perçage traversant (5) pouvant être aligné avec l'entaille (3), à travers lequel une autre vis peut être introduite.

2. Clou médullaire selon la revendication 1,
**caractérisé en ce que** le capuchon (4) présente un filetage femelle (8) et le clou médullaire un filetage mâle (2).

3. Clou médullaire selon la revendication 1,
**caractérisé en ce que** le capuchon présente une tige filetée mâle (12) et le clou médullaire un filetage femelle (13).

4. Clou médullaire selon l'une des revendications précédentes,
**caractérisé en ce que** l'entaille (3), et en conséquence le perçage traversant (5), est ovale, polygonal ou circulaire.

5. Clou médullaire selon l'une des revendications précédentes,
**caractérisé en ce que**, sur le capuchon, plus précisément sur le fond extérieur du capuchon, il est prévu un chapeau ou un épaulement qui présente une contre-dépouille (8).

6. Clou médullaire selon l'une des revendications précédentes,
**caractérisé en ce que** la face supérieure du capuchon possède un élément profilé (9, 10) servant au positionnement sans équivoque d'un instrument de visée ou au vissage.

7. Clou médullaire selon l'une des revendications précédentes,
**caractérisé en ce que** le capuchon (4), sur la face supérieure, possède un évidement (14) sous la forme d'une rainure servant au positionnement d'un instrument de visée ou au vissage du capuchon (4) sur le clou médullaire.
